# EUROPEAN PATENT APPLICATION

(11) **EP 4 570 196 A1**
(43) Date of publication of application: **18.06.2025**
(21) Application number: 24218077.6
(22) Date of filing: 06.12.2024
(51) Int. Cl.: A61B 17/32, A61B 17/00, A61B 17/29

(54) **MICRODEBRIDER WITH INNER TUBE RETENTION FEATURE**

(30) Priority: 11.12.2023 US 202363608331 P
(71) Applicant: Medtronic Xomed, LLC, Jacksonville, FL 32216 (US)
(72) Inventor: TURANO, Bo D., Jacksonville (US); FRIEND, Matthew J., Jacksonville (US)
(74) Representative: Viering, Jentschura & Partner mbB Patent- und Rechtsanwälte

(57) **Abstract**

A disposable cutting tool configured for use with a cutting device includes a shaft assembly that secures a proximal end of an outer shaft and an inner tube concentrically disposed within the outer shaft. An inner shaft supports a proximal hub connector at a proximal end thereof. A retainer is disposed atop the inner tube proximate a distal end of the proximal hub connector and cooperates with a locking flange of the shaft assembly to secure the inner tube therein. A seal is disposed between the locking flange and the retainer and prevents fluid from leaking from the shaft assembly. A shrink wrap is sealed atop the inner tube between the retainer and the proximal end of the outer shaft. Once assembled, the interference fit between the shrink wrap and the retainer prevents the inner tube from becoming unintentionally dislodged from the proximal hub connector during removal.

## Description

### FIELD

The present disclosure is generally directed to devices and systems for cutting and treating tissue, such as bone and soft tissue, and, more particularly, to microdebriders with removable handpieces with features to prevent the blades from becoming separated from the handpiece during removal.

### BACKGROUND

Devices and systems in accordance with the present disclosure may be suitable for a variety of procedures including ear, nose and throat (ENT) procedures, head and neck procedures, otology procedures, including otoneurologic procedures. Other surgical procedures for which the devices described herein are suitable include: mastoidectomies; nasopharyngeal and laryngeal procedures such as tonsillectomies, tracheal procedures, adenoidectomies, laryngeal lesion removal, and polypectomies; sinus procedures such as polypectomies, septoplasties, removals of septal spurs, antrostomies, frontal sinus trephination and irrigation, sinus opening, endoscopic DCR, correction of deviated septums and trans-sphenoidal procedures; rhinoplasty; and removal of fatty tissue in the maxillary and mandibular regions of the face.

Of particular significance is the usefulness of the devices and systems described herein with sinus surgery which is often challenging due to the location of the sinus cavity relative to sensitive organs such as the eyes and brain. Moreover, the relatively small size of the anatomy of interest to the surgeon and the complexity of the typical procedures places a heavy emphasis on precision. Examples of debriders with mechanical cutting components are described in commonly-owned U.S. Patent Nos.: 5,685,838; 5,957,881; and 6,293,957, the entire contents of each of which being incorporated by reference herein.

The Medtronic Straightshot^{®} M4/M5 Microdebriders or Medtronic Straightshot^{®} RAD40 or RAD60 Microdebriders, use sharp cutters to cut tissue, and suction to withdraw tissue. While tissue debridement with the Medtronic microdebrider range of devices is a simple and safe tissue treatment system, improvements can always be made to reduce the chances of operator error. For example, when removing straight blades from the Straightshot^{®} M4/M5, the inner tube is often left engaged within the handpiece due to the operator gripping the outer tube/hub assembly to remove the blade. As such, a need exists to reduce the chances of (or to prevent) the inner tube from being removed during removal of the handpiece.

### SUMMARY

Provided in accordance with the present disclosure is a disposable cutting tool configured for use with a cutting device for removing tissue or bone. The cutting tool includes an outer shaft having a cutting head at a distal end thereof configured to cut tissue or bone. A shaft assembly is configured to secure a proximal end of the outer shaft therein. An inner tube is concentrically disposed within the outer shaft and extends proximally therethrough. The inner shaft supports a proximal hub connector at a proximal end thereof. A retainer is disposed atop the inner tube proximate a distal end of the proximal hub connector, the retainer is configured to cooperate with a locking flange disposed on a proximal end of the shaft assembly to secure the inner tube therein. A seal is disposed between the locking flange and the retainer and is configured to prevent fluid from leaking from the shaft assembly. A shrink wrap (or other material to increase the outer diameter of the inner tube) is sealed atop the inner tube between the retainer and the proximal end of the outer shaft, wherein, once assembled, the interference fit between the shrink wrap and the retainer prevents the inner tube from becoming unintentionally dislodged from the proximal hub connector during removal.

In aspects according to the present disclosure, an inner diameter of the retainer is smaller than an outer diameter of the shrink wrap when sealed atop the inner tube.

In aspects according to the present disclosure, the distal end of the proximal hub connector is tapered and cooperates with the shrink wrap to capture the retainer.

In aspects according to the present disclosure, a proximal end of the proximal hub connector includes a universal mechanical interface which is adapted to connect to a locking collar disposed within the cutting device to secure the cutting tool therein.

In aspects according to the present disclosure, the retainer is mechanically secured to the shrink wrap after the retainer is engaged with the locking flange.

Provided in accordance with the present disclosure is a disposable cutting tool configured for use with a cutting device for removing tissue or bone. The cutting tool includes an outer shaft having a cutting head at a distal end thereof configured to cut tissue or bone. A shaft assembly is configured to secure a proximal end of the outer shaft therein. An inner tube is concentrically disposed within the outer shaft and extends proximally therethrough. The inner shaft supports a proximal hub connector at a proximal end thereof. A retainer is disposed atop the inner tube proximate a distal end of the proximal hub connector, and the retainer is configured to cooperate with a locking flange disposed on a proximal end of the shaft assembly to secure the inner tube therein. A shrink wrap (or other material to increase the outer diameter of the inner tube) is sealed atop the inner tube between the retainer and the proximal end of the outer shaft, the shrink wrap including seals on either end thereof, wherein, once assembled, the interference fit between the shrink wrap and the retainer prevents the inner tube from becoming unintentionally dislodged from the proximal hub connector during removal and wherein the seals on either end of the shrink wrap prevent fluid from leaking from the shaft assembly.

In aspects according to the present disclosure, an inner diameter of the retainer is smaller than an outer diameter of the shrink wrap when sealed atop the inner tube.

In aspects according to the present disclosure, the distal end of the proximal hub connector is tapered and cooperates with the shrink wrap to capture the retainer.

In aspects according to the present disclosure, a proximal end of the proximal hub connector includes a universal mechanical interface which is adapted to connect to a locking collar disposed within the cutting device to secure the cutting tool therein.

In aspects according to the present disclosure, the retainer is mechanically secured to the shrink wrap after the retainer is engaged with the locking flange.

Provided in accordance with the present disclosure is a method of assembling a disposable cutting tool configured for use with a cutting device for removing tissue or bone. The method includes: assembling, in order, atop a proximal end of an inner tube: a proximal hub connector; a retainer; a seal; and a shrink wrap (or other material to increase the outer diameter of the inner tube). The method also includes: sealing the shrink wrap atop the inner tube; inserting a distal end of the inner tube and the proximal hub connector, retainer, seal, and shrink wrap into a shaft assembly such that the distal end of the inner tube extends through an outer shaft; engaging the retainer within a proximal end of the shaft assembly to secure the inner tube therein; and mechanically securing the retainer to the shrink wrap to prevent the inner tube from becoming unintentionally dislodged from the proximal hub connector during removal.

In aspects according to the present disclosure, a locking flange engages the retainer within a proximal end of the shaft assembly to secure the inner tube therein.

In aspects according to the present disclosure, the inner tube is concentrically disposed within the outer shaft and extends to a distal end thereof.

In aspects according to the present disclosure, mechanically securing the retainer to the shrink wrap includes gluing, crimping, friction-fit, welding, or fusing.

### BRIEF DESCRIPTION OF THE DRAWINGS

In the drawings, where like numerals refer to like components throughout several views:
FIG. 1 is a perspective view of a surgical system in accordance with the present disclosure including a console and a powered surgical cutting device;
FIG. 2A is an internal, side view of a powered surgical cutting device for use with the system of FIG. 1 including a disposable cutting tool attached to a distal end thereof in accordance with one embodiment of the present disclosure;
FIG. 2B is an enlarged, internal side view of another embodiment of a disposable cutting tool for use with the surgical cutting device of FIG 1;
FIG. 3A is a side view of the cutting tool of FIG. 2A showing various component parts of a retainment assembly according to one embodiment of the present disclosure;
FIG. 3B is an internal, side view of the cutting tool of FIG. 2A showing the retainment assembly of FIG. 3A engaged;
FIG. 4A is a side view of the cutting tool of FIG. 2A showing various component parts of a retainment assembly according to another embodiment of the present disclosure; and
FIG. 4B is an internal, side view of the cutting tool of FIG. 2A showing the retainment assembly of FIG. 4A engaged.

### DETAILED DESCRIPTION

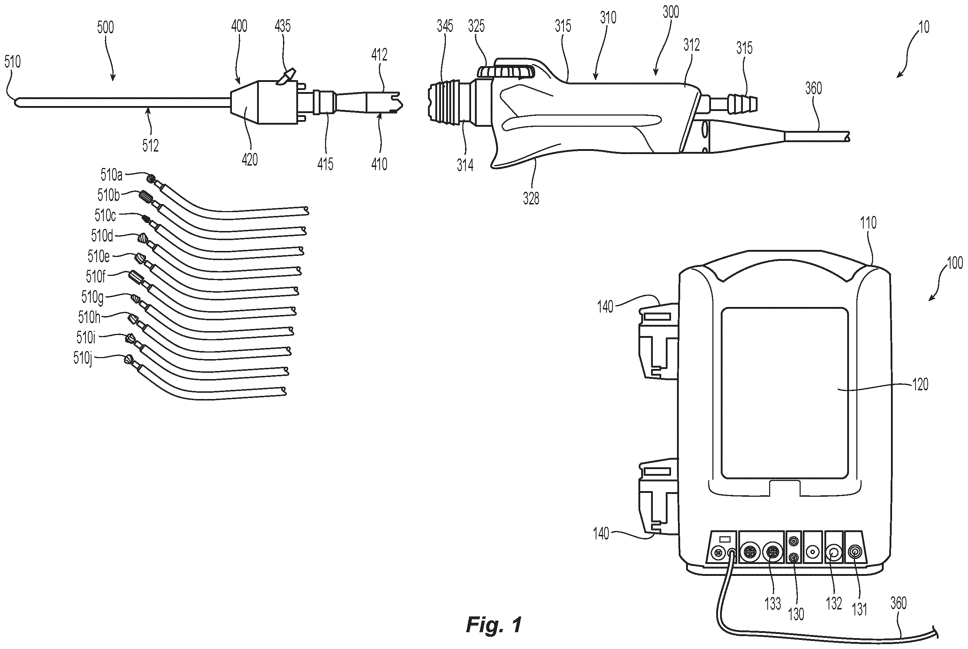

Turning to FIG. 1, a surgical system 10 provided in accordance with the present disclosure includes a console 100 and one or more surgical cutting devices 300. Console 100 may include an outer housing 110 enclosing the internal operable components of console 100, a touch screen graphical user interface (GUI) 120 to receive user input and display information to the user, a plurality of device ports 130-133, one or more fluid pumps 140, and/or other suitable features. One or more controllers including one or more processors and associated memory(s) are disposed within outer housing 110 and function: to provide power and control signals to devices connected to console 100; to process user inputs, feedback data, and other data received at console 100; and to control the one or more fluid pumps 140. Suitable hardware and drive mechanisms as part of or in addition to controller may be disposed within outer housing 110 to perform the various functions of console 100 and may include, for example, one or more central processing units (CPU's) and/or microcontroller units (MCU's), power generating and control hardware and corresponding firmware/software stored thereon, sensor circuitry, motors, pump drivers, pump controllers, etc.

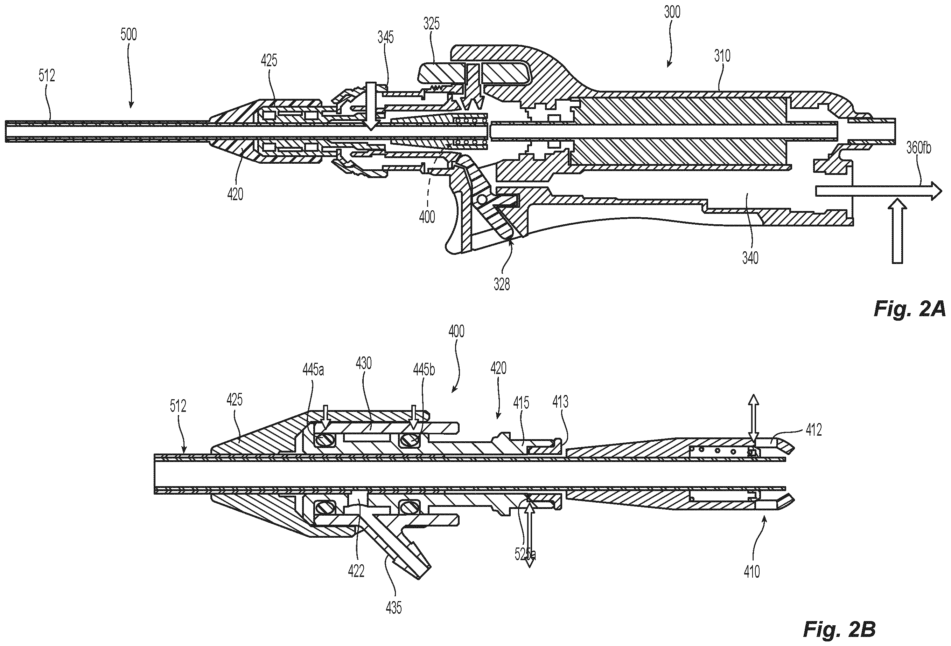

Turning now to FIGS. 1 and 2A, the one or more surgical cutting devices 300 may define any suitable configurations for use in performing various different surgical tasks, for use in various different procedures, etc. One example of a suitable surgical cutting device, e.g., surgical cutting device 300, generally includes a housing 315, including proximal and distal ends 312, 314 which together define a gripping area commonly referred to herein as handle 310. Proximal end 312 is configured to electrically interface and mechanically engage a terminal end of a power cord 360 that connects to the console 100 which powers and monitors the cutting device 300 and other components disposed therein.

The distal end 314 of the housing 315 includes a locking collar 345 which is configured to mechanically engage and lock a disposable cutting tool or blade 400 within the surgical cutting device 300 upon insertion therein. More particularly, the locking collar 345 is selectively movable between a first position facilitating insertion of the disposable cutting tool 400 within the distal end 314 of the housing 315 of the surgical device 300 and a second position wherein the locking collar 345 engages and locks a locking flange 415 disposed about the outer peripheral of the disposable cutting tool 400 within the housing 315 preventing removal of the disposable cutting tool 400 from the housing 315 until the locking collar 345 is moved back to the first position.

A locking mechanism 328 may be included to prevent accidental movement of the locking collar 345 during handling of the surgical cutting device 300. A rotating knob 325 may be included that operably couples to the outer shaft 512 and allows selective rotation of the disposable cutting tool 400 depending upon a particular purpose. Various examples of surgical cutting devices 300, cutting tools, rotating knobs 325, locking collars 345 are disclosed in commonly-owned U.S. Patent Application Serial No. 17/810,427 and U.S. Patent No. 10,881,427, the entire contents of both of which are incorporated by reference herein.

Disposable cutting tool 400 includes shaft assembly 420 which is configured to operably secure an outer shaft 512 of a surgical rotating or oscillating head 500, e.g., shaver or burr, having a specifically designed cutting head 510, rotational geometry, cutting geometry, angle relative the shaft 512 for accessing one or more surgical cavities (e.g., sinuses), etc. For the purposes herein, outer shaft 512 is generally described but includes the various internal inter-cooperating components operatively associated therewith. A proximal hub connector 410 of the disposable cutting tool 400 includes a proximal universal mechanical interface 412 which is operably configured to slidingly engage (e.g., via sliding receipt therein) the distal end 314 of housing 315 through locking collar 345 and mechanically and lock within housing 315 when locking collar 345 is disposed in the first position as described above (See FIG. 2A). Locking mechanism 328 may be moved into position to prevent accidental removal of the shaft assembly 420 once engaged. One or more alignment features (not shown) may be utilized to ensure proper alignment of the shaft assembly 420 prior to mechanical engagement within housing 315 or to insure rotational features of the shaft assembly 420, cutting device 300, and head 500 are aligned prior to activation. Again, examples are disclosed in commonly-owned U.S. Patent Application Serial No. 17/810,427 and U.S. Patent No. 10,881,427, the entire contents of both of which are incorporated by reference herein.

Shaft assembly 420 may also include an irrigation port 435 extending therefrom that is configured to operably connect to an irrigation source (not shown) configured to supply irrigation fluid to the distal end 510 of the head 500, e.g., shaver or burr.

Turning in particular to FIG. 2A, a motor 340 is disposed within handle 310 and is operably coupled via an array of mechanically-cooperating components (shown generically as components 350) to the proximal end mechanical interface 412 of the shaft assembly 420 to drive rotation and/or reciprocation of the shaft 512 to drive the head 510 to cut tissue or bone. Cord 360 connects motor 340 to console 100 to enable console 100 to power and control motor 340, thereby controlling the speed and other parameters of the shaft 512 as well as receiving feedback relating thereto. Motor 340 may be an electric motor, pneumatic motor, ultrasonic transducer, or other suitable motor configured to drive shaft 512 to rotate and/or reciprocate for cutting tissue or bone. Console 100 is configured to drive and control motor 340 such as, for example, a speed, torque, etc. output by motor 340. In aspects, surgical cutting device 300 may include additional features such as, for example, hand control(s), navigation, articulation, etc.

Turning briefly to FIG. 2B, an internal view of the shaft assembly 420 is shown highlighting a distal cap 425 disposed at the distal end of the shaft assembly 420 which is configured to house an irrigation hub 430 therein having an irrigation port 435 extending proximally therefrom. As mentioned above, irrigation port 435 connects to an irrigation source (not shown) configured to supply irrigation fluid to the distal head 510 of the shaver or burr 500 to act as a coolant or to clear surgical debris. Irrigation hub 430, in turn, is defined to secure the shaft assembly 420 therein atop a pair of O-rings or seals 445a and 445b on either side of a fluid communication passage 422 defined within the shaft assembly 420 configured to deliver fluid from the irrigation port 435 to the shaft 512. Irrigation fluid is delivered under pressure along an outer periphery of the shaft 512 through passage 422. Shaft assembly 420 also includes a locking flange 415 which is configured to mechanically engage the locking collar 345 as described above.

Outer shaft 512 may include one or more O-rings or seals disposed at various points therealong which are configured to facilitate mechanical engagement and/or provide a fluid-tight seal between the shaft 512 and the shaft assembly 420 during rotation. For example, the locking flange 415 operably couples to the proximal hub connector 410 of the shaft assembly 420 via a mechanical retainer 413. O-ring 525a provides a fluid-tight seal between retainer 413 and locking flange 415.

By equipping all of the different cutting heads 510a-510j with a proximal universal mechanical interface 412, the surgeon can quickly interchange heads 510a-510j as needed during the course of the operation. During a typical resection procedure, anywhere from two-to-five (2-5) different cutting heads 510a-510j may be employed and interchanged repeatedly over the course of the operation. As mentioned above, when changing cutting tools 400 from the handle 310, the inner tube 540 may remain engaged within the handle 310 especially if the operator grips the outer shaft 512 or shaft assembly 420 incorrectly to remove the cutting tool 400. This would require the operator to disassemble and reassembly the handle 310 or parts thereof to refit a new cutting tool 400 or substitute a new device 10.

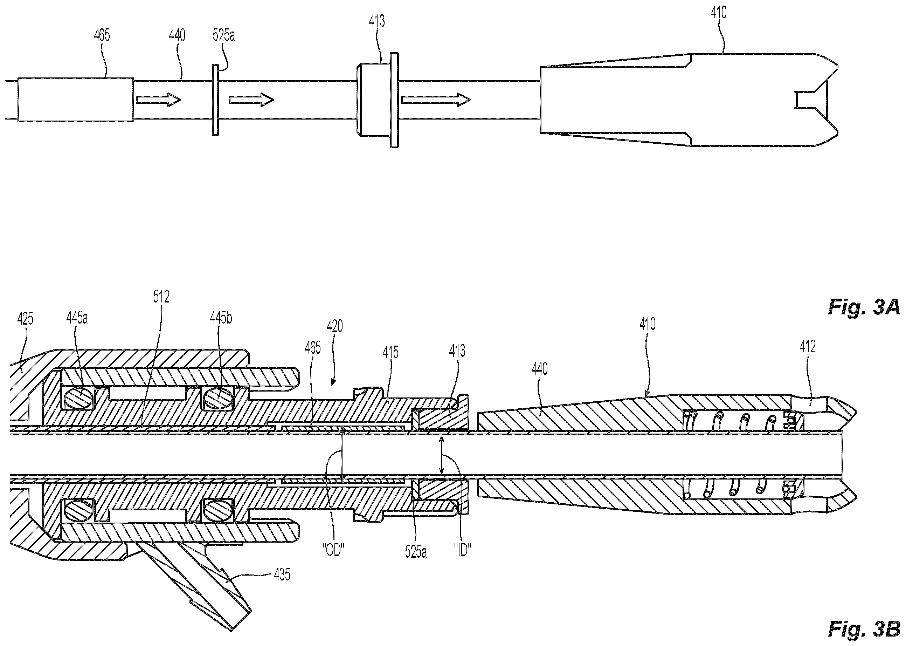

FIGS. 3A-3B show one proposed solution to prevent accidental disassembly of the proximal hub connector 410 and inner tube 440 when replacing cutting tools 400 which involves re-dimensioning components at specific interfaces to essentially mechanically lock the components after the cutting tool 400 is assembled thereby preventing the inner tube 440 from being pulled out from the proximal hub connector 410 once assembled. More particularly and as best shown in FIG. 3A, during assembly, the various component pieces of each cutting tool 400 are assembled atop the inner tube 440 in a particular order prior to mechanically interlocking the components thereon via gluing, shrink wrap, friction-fit, crimping, welding, or other ways known in the art. The proximal hub connector 410 is initially disposed at a proximal end of the inner tube 440. The retainer 413, seal 525a, and a shrink wrap 465 are then placed, in order, atop the inner tube 440 and move proximally towards the proximal hub connector 410. The inner diameter (ID) of the retainer 413 is dimensioned smaller than the outer diameter (OD) of the shrink wrap 465 even when shrunk such that the shrink wrap 465 maintains the retainer 413 in place atop the inner tube (FIG. 3B). Another material instead of shrink wrap 465 may be used to increase the outer diameter OD.

The proximal hub connector 410 and the assembled components, i.e., retainer 413, seal 525a, and shrink wrap 465, are then inserted into the shaft assembly 420 which is configured to secure the outer shaft 512 as described above. Once inserted into the shaft assembly 420, the retainer 413 is glued (or otherwise secured via crimping, friction-fit, welding, or fusing) into place to capture the shrink wrap 465 preventing the inner tube 440 from being removed from the proximal hub connector 410 (FIG. 3B) even if improperly handled. Seal 465 maintains fluid-tight integrity of the cutting tool 400 when assembled. The proximal hub connector 410 may include a tapered distal end 410a to facilitate capturing the retainer 413, e.g., in cooperation with the shrinking of the shrink wrap 465.

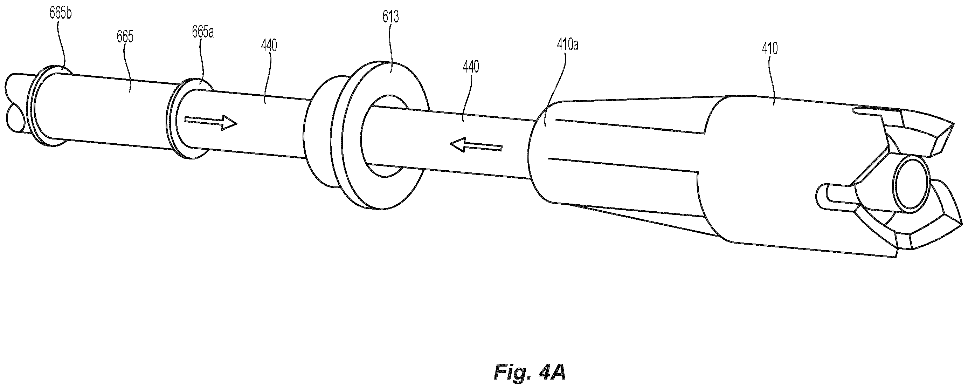

FIGS. 4A-4B show another proposed solution to prevent accidental disassembly of the proximal hub connector 410 and inner tube 440 when removing or replacing cutting tools 400 which involves both re-dimensioning components at specific interfaces to essentially mechanically lock the components after the cutting tool 400 is assembled and providing a shrink wrap 665 or other material with double seals. More particularly and as best shown in FIG. 4A, during assembly, the various component pieces of each cutting tool 400 are assembled atop the inner tube 440 in a particular order prior to mechanically interlocking the components thereon via gluing, shrink wrap, friction-fit, crimping, welding, or other ways known in the art. The proximal hub connector 410 is initially disposed at a proximal end of the inner tube 440 and pushed distally toward the retainer 613. The shrink wrap 665 is then placed atop the inner tube 440 and moved proximally towards the retainer 613 and the proximal hub connector 410. The proximal hub connector 410 may include a tapered distal end 410a to facilitate capturing the retainer 613, e.g., in cooperation with the shrinking of the shrink wrap 665.

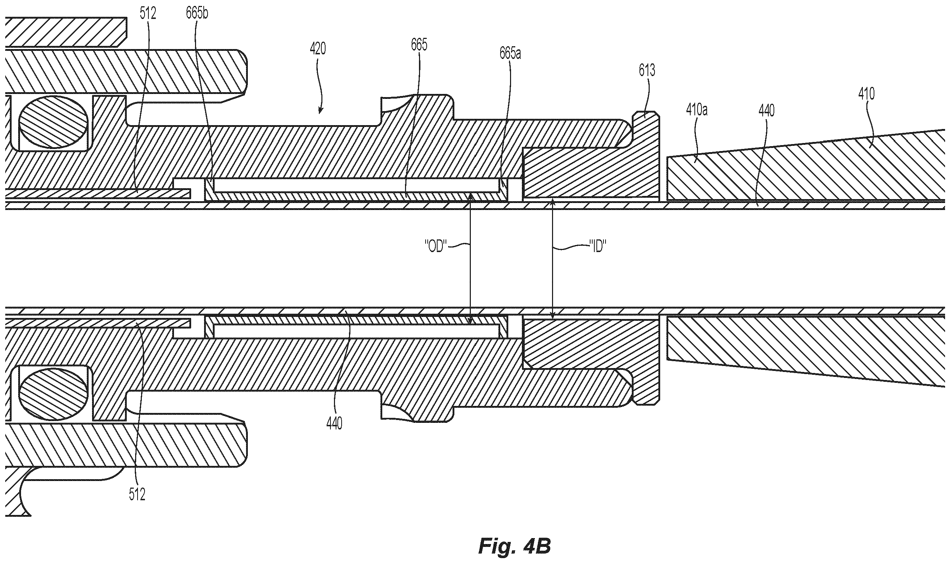

The inner diameter ID of the retainer 613 is dimensioned smaller than the outer diameter OD of the shrink wrap 665 even when shrunk such that the shrink wrap 665 maintains the retainer 613 in place atop the inner tube (FIG. 4B). The shrink wrap 665 includes proximal and distal seals 665a and 665b, respectively, that cooperate the maintain fluid integrity between the proximal hub connector 410 and the shaft assembly 420.

The proximal hub connector 410, retainer 613 and shrink wrap 665, are then inserted into the shaft assembly 420 which is configured to secure the outer shaft 512 as described above. Once inserted into the shaft assembly 420, the retainer 613 is glued into place to capture the shrink wrap 665 preventing the inner tube 440 from being removed from the proximal hub connector 410 (FIG. 3B) even if improperly handled. The proximal and distal seals 665a, 665b maintain fluid-tight integrity of the cutting tool 400 when assembled.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular configurations. It will be understood that various modifications may be made to the aspects and features disclosed herein. Therefore, the above description should not be construed as limiting, but merely as exemplifications of various aspects and features. Those skilled in the art will envision other modifications within the scope and spirit of the claims appended hereto.

For example, further embodiments may be as follows:
According to a 1st further embodiment, there is provided a disposable cutting tool configured for use with a cutting device for removing tissue or bone, comprising: an outer shaft including a cutting head at a distal end thereof configured to cut tissue or bone; a shaft assembly configured to secure a proximal end of the outer shaft therein; an inner tube concentrically disposed within the outer shaft and extending proximally therethrough, the inner shaft supporting a proximal hub connector at a proximal end thereof; a retainer disposed atop the inner tube proximate a distal end of the proximal hub connector, the retainer configured to cooperate with a locking flange disposed on a proximal end of the shaft assembly to secure the inner tube therein; and a shrink wrap sealed atop the inner tube between the retainer and the proximal end of the outer shaft, the shrink wrap including seals on either end thereof, wherein, once assembled, the interference fit between the shrink wrap and the retainer prevents the inner tube from becoming unintentionally dislodged from the proximal hub connector during removal and wherein the seals on either end of the shrink wrap prevent fluid from leaking from the shaft assembly.

According to a 2nd further embodiment, there is provided the disposable cutting tool according to the 1st further embodiment, wherein an inner diameter of the retainer is smaller than an outer diameter of the shrink wrap when sealed atop the inner tube.

According to a 3rd further embodiment, there is provided the disposable cutting tool according to the 1st or 2nd further embodiment, wherein the distal end of the proximal hub connector is tapered and cooperates with the shrink wrap to capture the retainer.

According to a 4th further embodiment, there is provided the disposable cutting tool according to one of the 1st to 3rd further embodiments, wherein a proximal end of the proximal hub connector includes a universal mechanical interface which is adapted to connect to a locking collar disposed within the cutting device to secure the cutting tool therein.

According to a 5th further embodiment, there is provided the disposable cutting tool according to one of the 1st to 4th further embodiments, wherein the retainer is mechanically secured to the shrink wrap after the retainer is engaged with the locking flange.

## Claims

1. A disposable cutting tool configured for use with a cutting device for removing tissue or bone, comprising:
an outer shaft including a cutting head at a distal end thereof configured to cut tissue or bone;
a shaft assembly configured to secure a proximal end of the outer shaft therein;
an inner tube concentrically disposed within the outer shaft and extending proximally therethrough, the inner shaft supporting a proximal hub connector at a proximal end thereof;
a retainer disposed atop the inner tube proximate a distal end of the proximal hub connector, the retainer configured to cooperate with a locking flange disposed on a proximal end of the shaft assembly to secure the inner tube therein;
a seal disposed between the locking flange and the retainer configured to prevent fluid from leaking from the shaft assembly; and
a shrink wrap sealed atop the inner tube between the retainer and the proximal end of the outer shaft, wherein, once assembled, the interference fit between the shrink wrap and the retainer prevents the inner tube from becoming unintentionally dislodged from the proximal hub connector during removal.

2. The disposable cutting tool according to claim 1, wherein an inner diameter of the retainer is smaller than an outer diameter of the shrink wrap when sealed atop the inner tube.

3. The disposable cutting tool according to claim 1 or 2, wherein the distal end of the proximal hub connector is tapered and cooperates with the shrink wrap to capture the retainer.

4. The disposable cutting tool according to one of claims 1-3, wherein a proximal end of the proximal hub connector includes a universal mechanical interface which is adapted to connect to a locking collar disposed within the cutting device to secure the cutting tool therein.

5. The disposable cutting tool according to one of claims 1-4, wherein the retainer is mechanically secured to the shrink wrap after the retainer is engaged with the locking flange.

6. A method of assembling a disposable cutting tool configured for use with a cutting device for removing tissue or bone, comprising:
assembling, in order, atop a proximal end of an inner tube:
a proximal hub connector;
a retainer;
a seal; and
a shrink wrap;
sealing the shrink wrap atop the inner tube;
inserting a distal end of the inner tube and the proximal hub connector, retainer, seal, and shrink wrap into a shaft assembly such that the distal end of the inner tube extends through an outer shaft;
engaging the retainer within a proximal end of the shaft assembly to secure the inner tube therein; and
mechanically securing the retainer to the shrink wrap to prevent the inner tube from becoming unintentionally dislodged from the proximal hub connector during removal.

7. The method according to claim 6, wherein a locking flange engages the retainer within a proximal end of the shaft assembly to secure the inner tube therein.

8. The method according to claim 6 or 7, wherein the inner tube is concentrically disposed within the outer shaft and extends to a distal end thereof.

9. The method according to one of claims 6-8, wherein mechanically securing the retainer to the shrink wrap includes gluing, crimping, friction-fit, welding, or fusing.
